# EUROPEAN PATENT APPLICATION

(11) **EP 4 272 790 A1**
(43) Date of publication of application: **08.11.2023**
(21) Application number: 20967887.9
(22) Date of filing: 31.12.2020
(51) Int. Cl.: A61M 16/00

(54) **RESPIRATORY VENTILATION METHOD AND DEVICE, ANESTHESIA MACHINE AND COMPUTER-READABLE STORAGE MEDIUM**

(71) Applicant: Shenzhen Mindray Animal Medical Technology Co., Ltd., Shenzhen, Guangdong 518110 (CN)
(72) Inventor: ZHOU, Xiaoyong, Shenzhen, Guangdong 518057 (CN); CHEN, Yiwei, Shenzhen, Guangdong 518057 (CN)
(74) Representative: KIPA AB
(86) International application number: PCT/CN2020/142459
(87) International publication number: WO 2022/141549

(57) **Abstract**

A respiratory ventilation method and device, an anesthesia machine and a computer-readable storage medium. The respiratory ventilation device comprises a patient-end respiratory system (1), a machine-end respiratory system (2), a flow monitor (3) and a processor (4), wherein at an inspiratory phase, first gas in an exhalation container (22) of the machine-end respiratory system (2) is driven to the patient-end respiratory system (1) by means of drive gas, and transmitted to a patient interface (10) by means of the patient-end respiratory system (1), and the first flow of the drive gas in the exhalation container (22) is detected by means of the flow monitor (3); at an expiratory phase, exhaled gas is received from the patient interface (10) and transmitted to the exhalation container (22) by means of the patient-end respiratory system (1), superfluous mixed gas in the patient-end respiratory system (1) is exhausted by means of an exhaust branch (23), and the second flow of mixed gas is detected by means of the flow monitor (3); and the processor (4) can calculate the tidal volume of one respiratory cycle on the basis of the first flow and the second flow.

## Description

### TECHNICAL FIELD

Embodiments of the disclosure relate to the technical field of medical instruments, and in particular, to a respiratory ventilation method and device, an anesthesia machine and a computer-readable storage medium.

### BACKGROUND

Currently, monitoring a patient's tidal volume during ventilation is an important function of anesthesia machines. According to different parts to be measured by the anesthesia machines, existing tidal volume monitoring solutions of the anesthesia machines are mainly classified into two types of solutions: patient-end monitoring and machine-end monitoring. A sensor used for the patient-end monitoring is placed very close to the patient's airway. The patient-end monitoring solution has the advantages of accurate measurement and high sensitivity because the sensor is close to the patient's airway. However, precisely because of the sensor being close to the patient's airway, the sensor is more likely affected by patient's airway secretions and exhaled breath condensate, the long-term accumulation of which would cause drift or deviation of the sensor, resulting in inaccurate tidal volume measurement, or even leading to failure of ventilation control and thus bringing about inaccurate tidal volume measurement.

### SUMMARY

Embodiments of the disclosure provide a respiratory ventilation method and device, an anesthesia machine and a computer-readable storage medium, which can improve the accuracy of tidal volume measurement.

The technical solutions of the embodiments of the disclosure may be implemented as follows.

The embodiments of the disclosure provide a respiratory ventilation device, including: a patient-end respiratory system, a machine-end respiratory system, a flow monitor and a processor.

The machine-end respiratory system includes: an intake branch at an inspiratory phase, a respiratory container, and an exhaust branch at an expiratory phase.

The intake branch and the exhaust branch are both connected to the respiratory container via the flow monitor, and the respiratory container is connected to the patient-end respiratory system.

The processor is connected to the flow monitor.

At the inspiratory phase, a first gas in the respiratory container is driven to the patient-end respiratory system by means of a drive gas transmitted in the intake branch, and is transmitted to a patient interface through the patient-end respiratory system, and a first flow of the drive gas in the respiratory container is measured by the flow monitor.

At the expiratory phase, an exhaled gas is received from the patient interface and is transmitted to the respiratory container through the patient-end respiratory system, a superfluous mixed gas in the respiratory container is exhausted through the exhaust branch, and a second flow of the mixed gas is measured by the flow monitor.

The processor is configured to calculate a tidal volume for one respiratory cycle on the basis of the first flow and the second flow.

In the respiratory ventilation device described above, the flow monitor is a bidirectional flow sensor.

In the respiratory ventilation device described above, the flow monitor is a one-way flow sensor; the flow monitor includes: a first flow sensor and a second flow sensor.

The first flow sensor is connected to the intake branch, and the second flow sensor is connected to the exhaust branch.

At the inspiratory phase, the first flow of the drive gas in the respiratory container is measured by the first flow sensor.

At the expiratory phase, the second flow of the mixed gas is measured by the second flow sensor.

The respiratory ventilation device described above further includes: a first one-way valve and a second one-way valve; the flow monitor is a one-way flow sensor; and the flow monitor includes: a first flow sensor and a second flow sensor.

The first one-way valve is configured to transmit the drive gas to the respiratory container.

The second one-way valve is configured to transmit the mixed gas to the exhaust branch.

The intake branch and the exhaust branch are both connected to the respiratory container via the first flow sensor, the first one-way valve, the second flow sensor and the second one-way valve; and the first one-way valve is connected to the first flow sensor, and the second one-way valve is connected to the second flow sensor.

At the inspiratory phase, the first flow of the drive gas in the respiratory container is measured by means of the first flow sensor and the first one-way valve.

At the expiratory phase, the second flow of the mixed gas is measured by means of the second flow sensor and the second one-way valve.

The respiratory ventilation device described above further includes: a gas delivery branch; the gas delivery branch is provided with a third flow sensor, a fresh gas interface and an evaporator.

At the inspiratory phase, a second gas is received through the fresh gas interface and is transmitted to the evaporator to bring a third gas out, and the third gas is transmitted to the patient interface through the patient-end respiratory system; a third flow of the third gas delivered through the gas delivery branch is measured by the third flow sensor.

At the expiratory phase, receiving a fourth gas through the fresh gas interface and transmitting the fourth gas to the evaporator to bring a fifth gas out, and transmitting the fifth gas to the respiratory container through the patient-end respiratory system; and a third flow of the fifth gas delivered through the gas delivery branch is measured by the third flow sensor.

The processor is configured to calculate a tidal volume for one respiratory cycle on the basis of the first flow, the second flow and the third flow.

In the respiratory ventilation device described above, the patient-end respiratory system further includes: an inspiratory branch and an expiratory branch.

The inspiratory branch and the expiratory branch are both connected to the respiratory container at one common connection end of the inspiratory branch and the expiratory branch, the gas delivery branch is connected to the inspiratory branch, and the patient interface is provided at the other common connection end of the inspiratory branch and the expiratory branch.

At the inspiratory phase, the drive gas is transmitted to the inspiratory branch through a drive gas interface, and the first gas in the respiratory container is driven to the inspiratory branch and is transmitted from the inspiratory branch to the patient interface.

At the expiratory phase, the exhaled gas is received from the patient interface and is transmitted to the respiratory container through the expiratory branch, and the superfluous mixed gas in the respiratory container is exhausted through the exhaust branch.

In the respiratory ventilation device described above, the respiratory container includes: a gas mask, a gas bag and a gas valve.

The gas bag is arranged in an inner cavity of the gas mask and is connected to the patient-end respiratory system, the gas bag is of a deformable structure, and the gas bag isolates the drive gas in the gas mask from the first gas in the gas bag.

The intake branch is connected to the gas mask, and the exhaust branch is connected to the gas bag via the gas valve.

At the inspiratory phase, the drive gas is transmitted to the gas mask through the intake branch, the gas bag shrinks and deforms under the action of a drive gas pressure in the gas mask, the first gas is compressed to be transmitted to the patient interface through the patient-end respiratory system, and the third gas is transmitted through the gas transmission branch and is transmitted to the patient interface through the patient-end respiratory system.

At the expiratory phase, the exhaled gas is received from the patient interface and is transmitted to the gas bag through the patient-end respiratory system, and the fifth gas is received from the gas delivery branch and is transmitted to the gas bag through the patient-end respiratory system.

The gas bag expands and deforms under the action of the fifth gas and the exhaled gas, the drive gas in the gas mask is compressed to be exhausted through the exhaust branch, and when a pressure in the gas bag is higher than a pressure threshold of the gas valve, the fifth gas and the exhaled gas in the gas bag are compressed to be exhausted through the exhaust branch.

In the respiratory ventilation device described above, the respiratory container includes: an exchange cavity.

The exchange cavity is of a hollow tubular structure, one end of the exchange cavity is connected to the intake branch and the exhaust branch, and the other end of the exchange cavity is connected to the patient-end respiratory system.

At the inspiratory phase, the drive gas is transmitted to the exchange cavity through the intake branch, the first gas in the exchange cavity is transmitted to the patient interface through the patient-end respiratory system under a pushing action of the drive gas pressure, and the third gas is transmitted through the gas transmission branch and is transmitted to the patient interface through the patient-end respiratory system.

At the expiratory phase, the exhaled gas is received from the patient interface and is transmitted to the exchange cavity through the patient-end respiratory system, and the fifth gas is received from the gas delivery branch and is transmitted to the exchange cavity through the patient-end respiratory system.

The fifth gas and the exhaled gas in the exchange cavity push the drive gas for mixing, to form the mixed gas, and the mixed gas is exhausted through the exhaust branch.

The embodiments of the disclosure further provides a respiratory ventilation method, which is applied to the respiratory ventilation device as described above. The method includes:
at an inspiratory phase, driving a first gas in a respiratory container to a patient-end respiratory system by means of a drive gas transmitted in an intake branch, and transmitting the first gas to a patient interface through the patient-end respiratory system;
measuring a first flow of the drive gas in the respiratory container by a flow monitor;
at an expiratory phase, receiving an exhaled gas from a patient interface and transmitting the exhaled gas to the respiratory container through the patient-end respiratory system, and exhausting a superfluous mixed gas from the respiratory container through an exhaust branch;
measuring a second flow of the mixed gas by the flow monitor; and
calculating a tidal volume for one respiratory cycle on the basis of the first flow and the second flow.

In the respiratory ventilation method described above, the flow monitor includes: a first flow sensor and a second flow sensor;
measuring a first flow of the drive gas in the respiratory container by a flow monitor includes:
measuring the first flow of the drive gas in the respiratory container by the first flow sensor; and
measuring a second flow of the mixed gas by the flow monitor includes:
measuring the second flow of the mixed gas by the second flow sensor.

In the respiratory ventilation method described above, calculating a tidal volume for one respiratory cycle on the basis of the first flow and the second flow includes:
obtaining the tidal volume for one respiratory cycle by using the first flow as an inspiratory tidal volume and using the second flow as an expiratory tidal volume.

In the respiratory ventilation method described above, calculating a tidal volume for one respiratory cycle on the basis of the first flow and the second flow includes:
obtaining a fresh gas flow in one respiratory cycle;
obtaining an inspiratory tidal volume by adding the first flow to the fresh gas flow; and
obtaining an expiratory tidal volume by subtracting the fresh gas flow from the second flow, and obtaining the tidal volume for one respiratory cycle by adding the inspiratory tidal volume to the expiratory tidal volume.

In the respiratory ventilation method described above, the respiratory ventilation device includes: a third flow sensor; obtaining a fresh gas flow in one respiratory cycle includes:
obtaining the fresh gas flow by dividing the subtraction of the first flow from the second flow by two; or
at the end of exhalation, using a third flow monitored by the third flow sensor as the fresh gas flow; or
receiving the fresh gas flow from a front-end interface; or
determining the fresh gas flow on the basis of a third flow measured by the third flow sensor and a functional relationship between a drive gas flow and the fresh gas flow.

In the respiratory ventilation method described above, the patient-end respiratory system includes a gas delivery branch; the method further includes:
at the inspiratory phase, receiving a second gas through a fresh gas interface in the gas delivery branch and transmitting the second gas to a evaporator in the gas delivery branch to bring a third gas out, and transmitting the third gas to the patient interface through the patient-end respiratory system;
measuring, by the third flow sensor, a third flow of the third gas delivered through the gas delivery branch;
at the expiratory phase, receiving a fourth gas through the fresh gas interface and transmitting the fourth gas to the evaporator to bring a fifth gas out, and transmitting the fifth gas to the respiratory container through the patient-end respiratory system; and
measuring, by the third flow sensor, a third flow of the fifth gas delivered through the gas delivery branch.

In the respiratory ventilation method described above, calculating a tidal volume for one respiratory cycle on the basis of the first flow and the second flow includes:
calculating a tidal volume for one respiratory cycle on the basis of the first flow, the second flow and the third flow.

In the respiratory ventilation method described above, calculating a tidal volume for one respiratory cycle on the basis of the first flow, the second flow and the third flow includes:
obtaining an inspiratory tidal volume by adding the first flow to the third flow;
obtaining an expiratory tidal volume by subtracting the third flow from the second flow; and
obtaining the tidal volume for one respiratory cycle by adding the inspiratory tidal volume to the respiratory tidal volume.

The embodiments of the disclosure further provide an anesthesia machine, including: the respiratory ventilation device as described above.

The embodiments of the disclosure further provide a computer-readable storage medium storing executable ventilation instructions configured to implement, when executed by a processor of an expiratory ventilation device, the respiratory ventilation method as described above.

In the embodiments of the disclosure, the flow monitor is arranged on the side of the machine-end respiratory system away from the patient-end respiratory system. At the inspiratory phase, the first gas in the respiratory container is driven to the patient-end respiratory system by means of the drive gas, and is transmitted to the patient interface through the patient-end respiratory system, and the first flow of the drive gas in the respiratory container is measured by the flow monitor; at the expiratory phase, an exhaled gas is received from the patient interface and is transmitted to the respiratory container through the patient-end respiratory system, a superfluous mixed gas in the respiratory container is exhausted through the exhaust branch, and a second flow of the mixed gas is measured by the flow monitor; and the processor may calculate the tidal volume on the basis of the first flow and the second flow. Since the flow monitor may monitor the gas flows in the machine-end respiratory system and the influence of different gas flows during inhalation and exhalation is taken into account, the tidal volume obtained by monitoring in this way has a high accuracy.

### BRIEF DESCRIPTION OF THE DRAWINGS

FIG. 1 is a first structural diagram of an exemplary respiratory ventilation device according to embodiments of the disclosure;
FIG. 2 is a second structural diagram of an exemplary respiratory ventilation device according to the embodiments of the disclosure;
FIG. 3 is a third structural diagram of an exemplary respiratory ventilation device according to the embodiments of the disclosure;
FIG. 4 is a fourth structural diagram of an exemplary respiratory ventilation device according to embodiments of the disclosure;
FIG. 5 is a fifth structural diagram of an exemplary respiratory ventilation device according to the embodiments of the disclosure;
FIG. 6 is a sixth structural diagram of an exemplary respiratory ventilation device according to the embodiments of the disclosure;
FIG. 7 is a seventh structural diagram of an exemplary respiratory ventilation device according to embodiments of the disclosure;
FIG. 8 is an eighth structural diagram of an exemplary respiratory ventilation device according to embodiments of the disclosure;
FIG. 9 is a first flow chart of an exemplary respiratory ventilation method according to embodiments of the disclosure;
FIG. 10 is a second flow chart of an exemplary respiratory ventilation method according to embodiments of the disclosure;
FIG. 11 is a third flow chart of an exemplary respiratory ventilation method according to embodiments of the disclosure;
FIG. 12 is a fourth flow chart of an exemplary respiratory ventilation method according to embodiments of the disclosure;
FIG. 13 is a fifth flow chart of an exemplary respiratory ventilation method according to embodiments of the disclosure;
FIG. 14 is a sixth flow chart of an exemplary respiratory ventilation method according to embodiments of the disclosure;
FIG. 15 is a seventh flow chart of an exemplary respiratory ventilation method according to embodiments of the disclosure;
FIG. 16 is an eighth flow chart of an exemplary respiratory ventilation method according to embodiments of the disclosure; and
FIG. 17 is a ninth flow chart of an exemplary respiratory ventilation method according to embodiments of the disclosure.

### DESCRIPTION OF THE PREFERRED EMBODIMENTS

The technical solutions in embodiments of the disclosure will be clearly and completely described below with reference to the accompanying drawings of the embodiments of the disclosure.

In order to understand the features and technical contents of embodiments of the disclosure in more detail, the implementation of the embodiments of the disclosure will be described below in detail with reference to the accompanying drawings, which are for reference and illustration only, and are not intended to limit the embodiments of the disclosure.

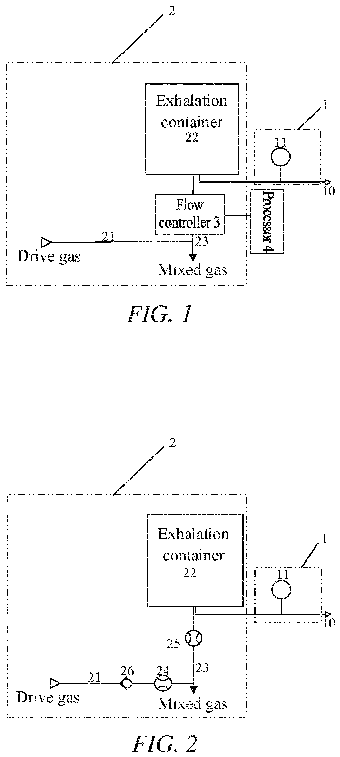

The embodiments of the disclosure provide a respiratory ventilation method, which is applied to a respiratory ventilation device. It should be noted that in the embodiments of the disclosure, the respiratory ventilation method may be implemented by the respiratory ventilation device or a respiratory ventilation apparatus. FIG. 1 is a first structural diagram of a respiratory ventilation device according to the embodiments of the disclosure.

As shown in FIG. 1, the respiratory ventilation device includes a patient-end respiratory system 1, a machine-end respiratory system 2, a flow monitor 3 and a processor 4.

The machine-end respiratory system 2 includes an intake branch 21 at an inspiratory phase, a respiratory container 22, and an exhaust branch 23 at an expiratory phase.

The intake branch 21 and the exhaust branch 23 are both connected to the respiratory container 22 via the flow monitor 3, and the respiratory container 22 is connected to the patient-end respiratory system 1.

The processor 4 is connected to the flow monitor 3.

At the inspiratory phase, a first gas in the respiratory container 22 is driven to the patient-end respiratory system 1 by means of a drive gas transmitted in the intake branch 21, and is transmitted to a patient interface 10 through the patient-end respiratory system 1. A first flow of the drive gas in the respiratory container 22 is measured by the flow monitor 3.

At the expiratory phase, an exhaled gas is received from the patient interface 10 and is transmitted to the respiratory container 22 through the patient-end respiratory system 1, and a superfluous mixed gas in the respiratory container 22 is exhausted through the exhaust branch 23. A second flow of the mixed gas is measured by the flow monitor 3.

The processor 4 is configured to calculate a tidal volume for one respiratory cycle on the basis of the first flow and the second flow.

In the embodiments of the disclosure, at the inspiratory phase, the intake branch 21 of the machine-end respiratory system 2 transmits the drive gas to the respiratory container 22. The drive gas drives the first gas in the respiratory container 22 to be transmitted to the patient interface 10 through the patient-end respiratory system 1 connected to the respiratory container 22. The patient interface 10 is connected to a patient, and the patient inhales the first gas through the patient interface 10. In the embodiments of the disclosure, the flow monitor 3 is a bidirectional flow sensor, and the flow monitor 3 measures the first flow of the drive gas. The flow monitor 3 transmits a value of the first flow to the processor 4, and the processor 4 may calculate an inspiratory tidal volume at the inspiratory phase on the basis of the first flow.

In the embodiments of the disclosure, at the expiratory phase, the exhaled gas from the patient is transmitted from the patient-end respiratory system 1 to the respiratory container 22 through the patient interface 10. The exhaled gas is injected into the respiratory container 22, and the mixed gas in the respiratory container 22 is compressed out of the respiratory container 22. The respiratory container 22 is connected to the exhaust branch 23, and the mixed gas is exhausted through the exhaust branch 23. In the process of exhausting the mixed gas through the exhaust branch 23, the flow monitor 3 measures the second flow of the superfluous mixed gas in the respiratory container 22. The flow monitor 3 transmits the second flow to the processor 4, and the processor 4 may calculate a respiratory tidal volume at the expiratory phase on the basis of the second flow. In the embodiments of the disclosure, the inspiratory tidal volume plus the expiratory tidal volume equals the tidal volume for one respiratory cycle.

In the embodiments of the disclosure, the processor 4 may be implemented by software, hardware, firmware or a combination thereof, and may use a circuit, a single or multiple application specific integrated circuits (ASIC), a single or multiple general integrated circuits, a single or multiple microprocessors, a single or multiple programmable logic devices, or a combination of the above-mentioned circuits or devices, or other suitable circuits or devices, so that the processor 4 can perform corresponding functions of the respiratory ventilation device. The flow monitor 3 may be a membrane flow sensor, a vane flow sensor, or a Karman vortex flow sensor, which is not limited in the embodiments of the disclosure.

In the embodiments of the disclosure, the patient-end respiratory system 1 may be further provided with a pressure sensor 11. The pressure sensor 11 is configured to measure a pressure of the gas in the patient-end respiratory system 1. When the pressure of the gas in the patient-end respiratory system 1 is too high, the pressure sensor 11 sends a signal of too high pressure to the processor 4, and the processor 4 sends out an alarm by means of other apparatuses.

In the embodiments of the disclosure, the first gas is a mixed gas of air and an anesthetic gas. The mixed gas may be a drive gas. The mixed gas may also be a mixed gas of the drive gas, an anesthetic gas and the exhaled gas.

In the embodiments of the disclosure, the drive gas may be air, and when the drive gas is input into the intake branch 21 of an expiratory ventilation device, it may be input according to a fixed ratio. The fixed ratio is determined according to requirements during actual ventilation, which is not limited by the embodiments of the disclosure.

It should be noted that in the embodiments of the disclosure, the processor 4 is connected to the flow monitor 3. In order to simplify the diagrams, the processor 4 is not shown in the diagrams of the following embodiments.

In the embodiments of the disclosure, the flow monitor 3 is arranged on one side of the machine-end respiratory system 2. At the inspiratory phase, the first gas in the respiratory container 22 is driven to the patient-end respiratory system 1 by means of the drive gas, and is transmitted to the patient interface 10 through the patient-end respiratory system 1, and the first flow of the drive gas in the respiratory container 22 is measured by the flow monitor 3. At the expiratory phase, the exhaled gas is received from the patient interface 10 and is transmitted to the respiratory container 22 through the patient-end respiratory system 1, the superfluous mixed gas in the respiratory container 22 is exhausted through the exhaust branch 23, and the second flow of the mixed gas is measured by the flow monitor 3. The processor 4 may calculate the tidal volume on the basis of the first flow and the second flow, and since the flow monitor 3 is provided at the machine respiratory device end and the influence of different gas flows during inhalation and exhalation is taken into account, the tidal volume obtained by monitoring in this way has a high accuracy.

In some embodiments of the disclosure, as shown in FIG. 2, the flow monitor 3 includes a first flow sensor 24 and a second flow sensor 25. The first flow sensor 24 and the second flow sensor 25 are one-way flow sensors. The first flow sensor 24 is connected to the intake branch 21, and the second flow sensor 25 is connected to the exhaust branch 23. At the inspiratory phase, the first flow of the drive gas in the respiratory container 22 is measured by the first flow sensor 24. At the expiratory phase, the second flow of the superfluous mixed gas in the respiratory container is measured by the second flow sensor 25. The first flow sensor 24 transmits a value of the first flow to the processor 4, and the second flow sensor 25 transmits a value of the second flow to the processor 4. The processor 4 may calculate the inspiratory tidal volume at the inspiratory phase on the basis of the first flow. The processor 4 may calculate the expiratory tidal volume at the expiratory phase on the basis of the second flow. In the embodiments of the disclosure, the inspiratory tidal volume plus the expiratory tidal volume equals the tidal volume for one respiratory cycle.

In some embodiments of the disclosure, the intake branch 21 is further provided with a fifth one-way valve 26. The fifth one-way valve 26 enables the drive gas to flow only to the respiratory container 22.

It should be noted that the arrangement position of the first flow sensor 24 on the intake branch 21 is not limited in the embodiments of the disclosure, and the arrangement position of the second flow sensor 25 on the exhaust branch 23 is not limited in the embodiments of the disclosure. FIG. 2 merely shows an exemplary arrangement.

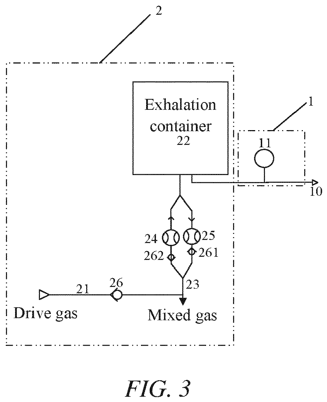

In some embodiments of the disclosure, as shown in FIG. 3, the respiratory ventilation device further includes a first one-way valve 262 and a second one-way valve 261. The flow monitor 3 includes a first flow sensor 24 and a second flow sensor 25. The first flow sensor 24 and the second flow sensor 25 are both one-way flow sensors. The first one-way valve 262 is configured to transmit the drive gas to the respiratory container 22. The second one-way valve 261 is configured to transmit the mixed gas to the exhaust branch 23. The intake branch 21 and the exhaust branch 23 22 are both connected to the respiratory container via the first flow sensor 24, the first one-way valve 262, the second flow sensor 25 and the second one-way valve 261. The first one-way valve 262 is connected to the first flow sensor 24, and the second one-way valve 261 is connected to the second flow sensor 25.

At the inspiratory phase, the drive gas flows to the respiratory container 22 through the first one-way valve 262 and the first flow sensor 24, and the first flow sensor 24 connected to the first one-way valve 262 measures the first flow of the drive gas in the respiratory container 22. At the expiratory phase, the superfluous mixed gas in the exhalation container 22 is exhausted from the exhaust branch through the second one-way valve 261 and the second flow sensor 25. The second flow sensor 25 measures the second flow of the mixed gas exhausted through the exhaust branch 23. The first flow sensor 24 transmits a value of the first flow to the processor 4, and the second flow sensor 25 transmits a value of the second flow to the processor 4. Similarly, the processor 4 may calculate the inspiratory tidal volume at the inspiratory phase on the basis of the first flow. The processor 4 may calculate the respiratory tidal volume at the expiratory phase on the basis of the second flow. In the embodiments of the disclosure, the inspiratory tidal volume plus the expiratory tidal volume equals the tidal volume for one respiratory cycle.

It should be noted that the arrangement positions of the first flow sensor 24, the first one-way valve 262, the second flow sensor 25 and the second one-way valve 261 on the intake branch 21 and the exhaust branch 23 are not limited in the disclosure. FIG. 3 merely shows an exemplary arrangement.

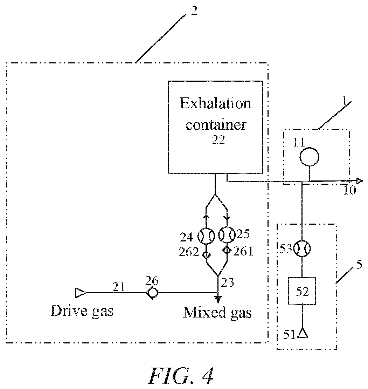

In some embodiments of the disclosure, as shown in FIG. 4, the respiratory ventilation device further includes a gas delivery branch 5. The gas delivery branch 5 is provided with a third flow sensor 53, a fresh gas interface 51 and an evaporator 52.

At the inspiratory phase, the second gas is received through the fresh gas interface 51 and is transmitted to the evaporator 52, and the second gas brings the third gas out of the evaporator 52. The third gas is transmitted to the patient interface 10 through the patient-end respiratory system 1. The third flow of the third gas delivered through the gas delivery branch 5 is measured by the third flow sensor 53.

At the expiratory phase, a fourth gas is received through the fresh gas interface 51 and is transmitted to the evaporator 52, and the fourth gas brings the fifth gas out of the evaporator 52. The fifth gas is transmitted to the respiratory container 22 through the patient-end respiratory system 1. The third flow of the fifth gas delivered through the gas delivery branch 1 is measured by the third flow sensor 53. The third flow sensor 53 transmits the values of the third flow of the third gas and the third flow of the fifth gas to the processor 4. The processor 4 calculates the tidal volume for one respiratory cycle on the basis of the first flow, the second flow and the third flow.

In the embodiments of the disclosure, at the inspiratory phase, the intake branch 21 of the machine-end respiratory system 2 transmits the drive gas to the respiratory container 22. The drive gas drives the first gas in the respiratory container 22 to be transmitted to the patient interface 10 through the patient-end respiratory system 1 connected to the respiratory container 22, and the patient inhales the first gas through the patient interface 10. At the same time, the second gas is received through the fresh gas interface 51 and is transmitted to the evaporator 52, the second gas brings the third gas out of the evaporator 52, and the third gas is transmitted to the patient interface 10 through the patient-end respiratory system 1. In the embodiments of the disclosure, the first flow of the drive gas entering the respiratory container 22 is measured by the first flow sensor 24. The third flow of the third gas delivered through the gas delivery branch 5 is measured by the third flow sensor 53. The first flow sensor 24 sends the value of the first flow to the processor 4, and the third flow sensor 53 sends the value of the third flow of the third gas to the processor 4. The processor 4 may calculate the inspiratory tidal volume at the inspiratory phase on the basis of the first flow and the third flow. For example, the processor 4 may calculate the inspiratory tidal volume at the inspiratory phase by adding the first flow to the third flow.

In the embodiments of the disclosure, at the expiratory phase, the exhaled gas from the patient is transmitted from the patient-end respiratory system 1 to the respiratory container 22 through the patient interface 10. At the same time, the gas delivery branch 5 receives a fourth gas through the fresh gas interface 51 and transmits the fourth gas to the evaporator 52, the fourth gas brings a fifth gas out of the evaporator 52, and the fifth gas is transmitted to the respiratory container 22 through the patient-end respiratory system 1. The exhaled gas and the fifth gas simultaneously compress the mixed gas in the respiratory container 22 out of the respiratory container 22. If the respiratory container 22 is of a structure in which a gas bag is provided inside a gas mask, the exhaled gas is injected into the gas bag, the gas bag expands, and the drive gas outside the gas bag and inside the gas mask is compressed out of the respiratory container and is exhausted through the exhaust branch 23. When a gas pressure in the gas bag reaches a certain level, the superfluous mixed gas in the gas bag is exhausted through the exhaust branch 23. If the exhalation container 22 is of a hollow tubular structure, the exhaled gas and the fifth gas compress the superfluous mixed gas in the exhalation container 22 out of the respiratory container 22, and the superfluous mixed gas is exhausted from the exhaust branch 23. The machine-end respiratory system 2 measures the second flow of the mixed gas exhausted from the exhaust branch 23 by the second flow sensor 25. The third flow of the fifth gas delivered through the gas delivery branch 5 is measured by the third flow sensor 53. The second flow sensor 25 sends the value of the second flow to the processor 4, and the third flow sensor 53 sends the value of the third flow of the fifth gas to the processor 4. The processor 4 may calculate the expiratory tidal volume at the expiratory phase by subtracting the third flow from the second flow.

It should be noted that at the inspiratory phase, the processor 4 obtains the inspiratory tidal volume at the inspiratory phase by adding the first flow to the third flow of the third gas. At the expiratory phase, the processor 4 obtains the expiratory tidal volume at the expiratory phase by subtracting the third flow of the fifth gas from the second flow. In the embodiments of the disclosure, the inspiratory tidal volume plus the expiratory tidal volume equals the tidal volume for one respiratory cycle.

In the embodiments of the disclosure, the second gas and the fourth gas are air or oxygen. The third gas and the fifth gas are air or a mixed gas of oxygen and an anesthetic gas, which is not limited in the embodiments of the disclosure.

In the embodiments of the disclosure, the third flow sensor 53 may be omitted from the respiratory ventilation device, and the processor 4 may calculate the third flow by means of the first flow sensor 24 and the second flow sensor 25. The processor 4 may obtain the third flow by dividing the subtraction of the first flow of the first flow sensor 24 at the inspiratory phase from the second flow of the second flow sensor 25 at the expiratory phase by two. In the embodiments of the disclosure, the processor 4 may obtain the third flow by dividing the subtraction of the first flow of the first flow sensor 24 at the inspiratory phase from the second flow of the second flow sensor 25 at the expiratory phase by 2.

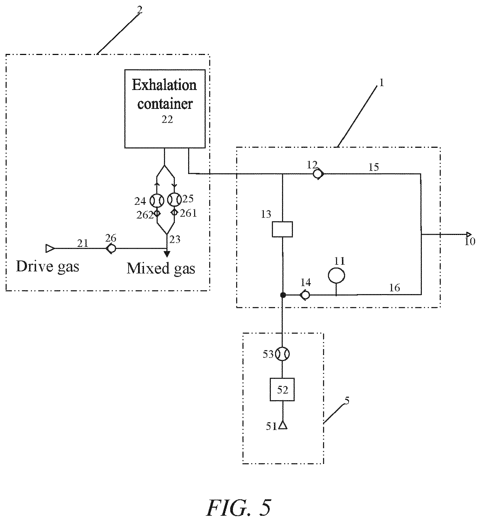

In some embodiments of the disclosure, as shown in FIG. 5, the patient-end respiratory system 1 further includes an inspiratory branch 16 and an expiratory branch 15. The inspiratory branch 16 and the expiratory branch 15 are both connected to the respiratory container 22 at one common connection end of the inspiratory branch 16 and the expiratory branch 15. The gas delivery branch 5 is connected to the inspiratory branch 16, and the patient interface 10 on the patient-end respiratory system 1 is provided at the other common connection end of the inspiratory branch 16 and the expiratory branch 15.

At the inspiratory phase, the drive gas is suctioned through the intake branch 21 and is transmitted into the respiratory container 22 through the intake branch 21. The drive gas drives the first gas in the respiratory container 22 to the inspiratory branch 16. The first gas is transmitted from the inspiratory branch 16 to the patient interface 10.

At the expiratory phase, the exhaled gas is received from the patient interface 10 and is transmitted to the respiratory container 22 through the expiratory branch 15. The exhaled gas compresses the superfluous mixed gas in the respiratory container 22, and the superfluous mixed gas is exhausted from the exhaust branch 23 connected to the respiratory container 22.

In the embodiments of the disclosure, at the inspiratory phase, the intake branch 21 of the machine-end respiratory system 2 transmits the drive gas to the respiratory container 22. The drive gas drives the first gas in the respiratory container 22 to be transmitted to the patient interface 10 through the inspiratory branch 16 connected to the respiratory container 22, and the patient inhales the first gas through the patient interface 10. At the same time, the second gas is received through the fresh gas interface 51 and is transmitted to the evaporator 52, the second gas brings the third gas out of the evaporator 52, and the third gas is transmitted to the patient interface 10 through the inspiratory branch 16. In the embodiments of the disclosure, the first flow sensor 24 measures the first flow of the drive gas in the respiratory container 22. The third flow sensor 53 measures the third flow of the third gas delivered through the gas delivery branch 5. The first flow sensor 24 sends the value of the first flow to the processor 4, and the third flow sensor 53 sends the value of the third flow of the third gas to the processor 4. The processor 4 may calculate the inspiratory tidal volume at the inspiratory phase by adding the first flow to the third flow.

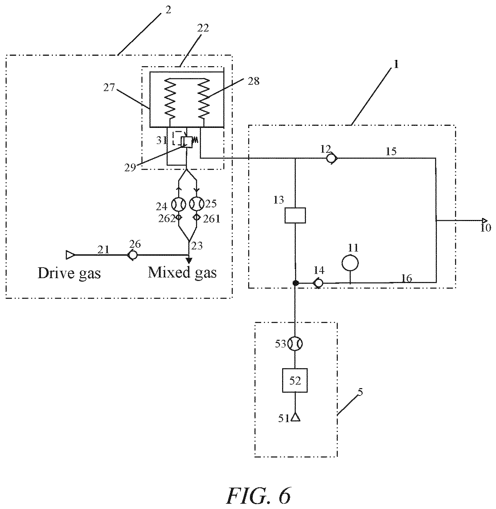

In the embodiments of the disclosure, at the expiratory phase, the exhaled gas from the patient is transmitted from the expiratory branch 15 to the respiratory container 22 through the patient interface 10. For example, the exhaled gas compresses the drive gas, from the gas mask 27 (shown in FIG. 6) of the respiratory container 22, out of the respiratory container 22. The respiratory container 22 is connected to the exhaust branch 23, and the superfluous mixed gas in the gas bag 28 (shown in FIG. 6) of the respiratory container 22 is also exhausted from the exhaust branch 23 by means of the compression from the exhaled gas. At the same time, the gas delivery branch 5 receives a fourth gas through the fresh gas interface 51 and transmits the fourth gas to the evaporator 52, the fourth gas brings a fifth gas out of the evaporator, and the fifth gas is transmitted to the respiratory container 22 through the inspiratory branch 16. The patient-end respiratory system 2 measures the second flow of the mixed gas exhausted from the exhaust branch 23 by the second flow sensor 25. The third flow of the fifth gas delivered through the gas delivery branch 5 is measured by the third flow sensor 53. The second flow sensor 25 sends the value of the second flow to the processor 4, and the third flow sensor 53 sends the value of the third flow of the fifth gas to the processor 4. The processor 4 may calculate the expiratory tidal volume at the expiratory phase by subtracting the third flow of the fifth gas from the second flow.

In the embodiments of the disclosure, since the gas delivery branch 5 delivers the third gas and the fifth gas at the inspiratory phase and at the expiratory phase, the flows of the third gas and the fifth gas are the same and are both the third gas flow.

It should be noted that the processor 4 obtains the inspiratory tidal volume at the inspiratory phase by adding the first flow to the third flow of the third gas at the inspiratory phase. The processor 4 obtains the expiratory tidal volume at the expiratory phase by subtracting the third flow of the fifth gas from the second flow at the expiratory phase. In the embodiments of the disclosure, the inspiratory tidal volume plus the expiratory tidal volume equals the tidal volume for one respiratory cycle.

It should be noted that the inspiratory branch 16 may be provided with a filter 13. At the inspiratory phase, the filter 13 is configured to filter impurities from the first gas, and at the expiratory phase, the filter 13 is configured to filter impurities from the fifth gas. The inspiratory branch 16 is further provided with a third one-way valve 14, and the expiratory branch 15 is further provided with a fourth one-way valve 12. The third one-way valve 14 enables the gas in the inspiratory branch 16 to be transmitted only towards the patient interface 10. The fourth one-way valve 12 enables the gas in the expiratory branch 15 to be transmitted only from the patient interface 10 to the respiratory container 22.

In some embodiments of the disclosure, as shown in FIG. 6, the respiratory container 22 includes a gas mask 27, a gas bag 28 and a gas valve 29. The gas bag 28 is arranged in an inner cavity of the gas mask 27, and the gas bag 28 is connected to the patient-end respiratory system 1. The gas bag 28 is of a deformable structure, and the gas bag 28 isolates the drive gas in the gas mask 27 from the first gas in the gas bag; the intake branch 21 is connected to the gas mask 27, and the exhaust branch 23 is connected to the gas bag 28 via the gas valve 29; and the intake branch 21 is connected to the gas mask 27, and the exhaust branch 23 is connected to the gas bag 28 via the gas valve 29.

At the inspiratory phase, the drive gas is transmitted to the gas mask 27 through the intake branch 21, the gas bag 28 shrinks and deforms under the action of a drive gas pressure in the gas mask 27, the first gas is compressed to be transmitted to the patient interface 10 through the patient-end respiratory system 1, and the third gas is transmitted through the gas transmission branch 5 and is transmitted to the patient interface 10 through the patient-end respiratory system 1.

At the expiratory phase, the exhaled gas is received from the patient interface 10 and is transmitted to the gas bag 28 through the patient-end respiratory system 1, and the fifth gas is received from the gas delivery branch 5 and is transmitted to the gas bag 28 through the patient-end respiratory system 1.

The gas bag 28 expands and deforms under the action of the fifth gas and the exhaled gas, the drive gas in the gas mask 27 is compressed to be exhausted through the exhaust branch 23, and when a pressure in the gas bag 28 is higher than a pressure threshold of the gas valve, the fifth gas and the exhaled gas in the gas bag 28 are compressed to be exhausted through the exhaust branch 23.

In the embodiments of the disclosure, at the inspiratory phase, the intake branch 21 of the machine-end respiratory system 2 transmits the drive gas into the gas mask of the respiratory container 22. The gas bag 28 shrinks and deforms under the action of the drive gas pressure in the gas mask 27 to compress the first gas. The first gas is transmitted to the patient interface 10 through the inspiratory branch 16 connected to the gas bag 28, and the patient inhales the first gas through the patient interface 10. At the same time, the second gas is received through the fresh gas interface 51 and is transmitted to the evaporator 52, the second gas brings the third gas out of the evaporator 52, and the third gas is transmitted to the patient interface 10 through the inspiratory branch 16. In the embodiments of the disclosure, the machine-end respiratory system 2 measures the first flow of the drive gas in the exhalation container 22 by the first flow sensor 24. The third flow of the third gas delivered through the gas delivery branch 5 is measured by the third flow sensor 53. The first flow sensor 24 sends the value of the first flow to the processor 4, and the third flow sensor 53 sends the value of the third flow of the third gas to the processor 4. The processor 4 may calculate the inspiratory tidal volume at the inspiratory phase on the basis of the first flow and the third flow.

In the embodiments of the disclosure, at the expiratory phase, the exhaled gas from the patient is transmitted from the expiratory branch 15 to the gas bag 28 of the respiratory container 22 through the patient interface 10. At the same time, the fourth gas is received through the fresh gas interface 51 and is transmitted to the evaporator 52, the fourth gas brings the fifth gas out of the evaporator 52, and the fifth gas is transmitted to the gas bag 28 through the inspiratory branch 16. The gas bag 28 expands under the action of the exhaled gas and the fifth gas, the drive gas in the gas mask 27 of the respiratory container 22 is first compressed into the exhaust branch 23 from a connection branch 31 connected to the gas mask 27, and the drive gas is exhausted from the gas mask 27 through the exhaust branch 23. When the gas bag 28 expands to the top of the gas mask 27 and a gas pressure therein is greater than the pressure threshold of the gas valve 29, the exhaled gas and the fifth gas in the gas bag 28 are exhausted through the exhaust branch 23 connected to the gas valve 29. The second flow of the mixed gas exhausted from the exhaust branch 23 is measured by the second flow sensor 24. The mixed gas includes the fifth gas, the drive gas and the exhaled gas. The third flow of the fifth gas delivered through the gas delivery branch 5 is measured by the third flow sensor 53. The second flow sensor 25 sends the value of the second flow to the processor 4, and the third flow sensor 53 sends the value of the third flow of the fifth gas to the processor 4. The processor may calculate the expiratory tidal volume at the expiratory phase on the basis of the second flow and the third flow.

In the embodiments of the disclosure, the processor 4 may measure the third flow at the end of the expiratory phase because the patient's exhalation has completely ended at the end of the expiratory phase. In this case, the gas bag 28 has risen to the top, after the fifth gas continuing to enter the gas bag 28 is mixed with the gas in the gas bag 28, and when the pressure in the gas bag 28 is greater than the pressure threshold of the gas valve 29, the mixed gas in the gas bag 28 overflows from the gas valve 29. Therefore, at the end of exhalation, the third flow can be obtained by monitoring data of the second flow sensor 25 at the respiratory container 22 in a point measurement manner.

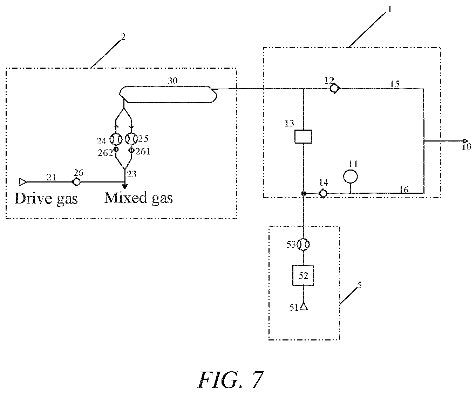

In some embodiments of the disclosure, as shown in FIG. 7, the respiratory container 22 includes an exchange cavity 30. The exchange cavity is of a hollow tubular structure, one end of the exchange cavity 30 is connected to the intake branch 21 and the exhaust branch 23, and the other end of the exchange cavity 30 is connected to the patient-end respiratory system 1. At the inspiratory phase, the drive gas is transmitted to the exchange cavity 30 through the intake branch 21, and the first gas in the exchange cavity 30 is transmitted to the patient interface 10 through the patient-end respiratory system 1 under the action of the drive gas pressure. At the same time, the third gas is transmitted through the gas transmission branch 5 and is transmitted to the patient interface 10 through the patient-end respiratory system 1. At the expiratory phase, the exhaled gas is received from the patient interface 10 and is transmitted to the exchange cavity 30 through the patient-end respiratory system 1, and the fifth gas is received from the gas delivery branch 5 at the same time and is transmitted to the exchange cavity 30 through the patient-end respiratory system 1. The exchange cavity 30 exhausts the superfluous mixed gas through the exhaust branch 23 under the pressure of the fifth gas and the exhaled gas.

In the embodiments of the disclosure, the exchange cavity is of a hollow linear tubular structure.

It should be noted that the exhausted mixed gas may include the exhaled gas, the fifth gas and the drive gas. The exhausted mixed gas may alternatively include the drive gas.

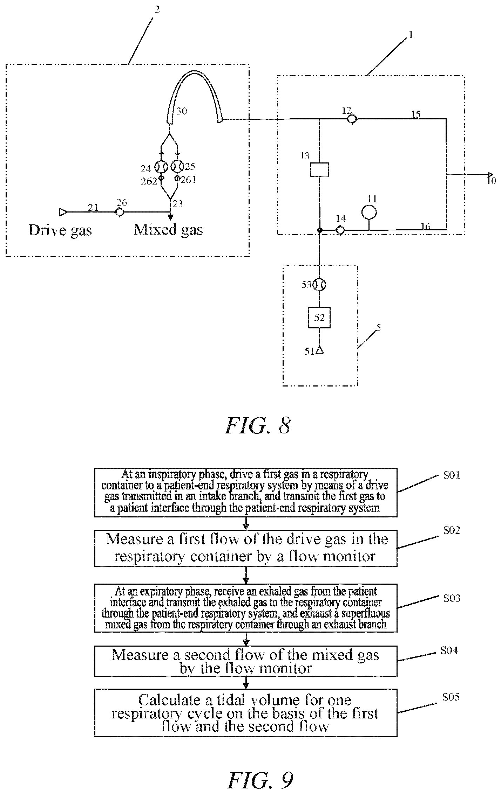

In some embodiments of the disclosure, as shown in FIG. 8, the respiratory container 22 includes an exchange cavity 30. The exchange cavity is of a hollow tubular structure having an inverted U shape. In other embodiments, the exchange cavity may also be of a hollow tubular structure having other shapes.

In the embodiments of the disclosure, the first flow sensor 24 and the second flow sensor 25 are arranged on one side of the machine-end respiratory system 2, and the first flow sensor 24 and the second flow sensor 25 are away from the patient. At the inspiratory phase, the first gas in the respiratory container 22 is driven to the patient-end respiratory system 1 by means of the drive gas, and is transmitted to the patient interface 10 through the patient-end respiratory system 1, and the first flow of the drive gas in the respiratory container 22 is measured by the first flow sensor 24. At the same time, the third gas is transmitted through the gas transmission branch 5 and is transmitted to the patient interface 10 through the patient-end respiratory system 1, and the third flow of the third gas is measured by the third flow sensor 53. At the expiratory phase, the exhaled gas is received from the patient interface 10 and is transmitted to the respiratory container 22 through the patient-end respiratory system, and the fifth gas is received from the gas delivery branch 5 at the same time and is transmitted to the respiratory container 22 through the patient-end respiratory system 1. Under the action of the fifth gas and the exhaled gas for the respiratory container 22, the superfluous mixed gas in the respiratory container 22 is exhausted through the exhaust branch 23, the second flow of the exhausted mixed gas is measured by the second flow sensor 25, and a fourth flow of the fifth gas delivered through the gas delivery branch 5 is measured by the third flow sensor 53. The processor 4 may calculate the tidal volume on the basis of the first flow, the second flow, the third flow and the fourth flow. Since the first flow sensor 24 and the second flow sensor 25 are arranged in the machine-end respiratory system 2 and the influence of different gas flows during the inhalation and exhalation is taken into account, the tidal volume obtained by monitoring in this way has a high accuracy.

On the basis of the structure of the respiratory ventilation device described above, FIG. 9 is a schematic flow chart of a respiratory ventilation method applied to a respiratory ventilation device according to the embodiments of the disclosure. As shown in FIG. 9, the respiratory ventilation method mainly includes the following steps.

At S01, at an inspiratory phase, a first gas in a respiratory container is driven to a patient-end respiratory system by means of a drive gas transmitted in an intake branch, and is transmitted to a patient interface through the patient-end respiratory system.

In the embodiments of the disclosure, at the inspiratory phase, the intake branch transmits the drive gas to the respiratory container. The drive gas drives the first gas in the respiratory container to be transmitted to the patient interface through the patient-end respiratory system connected to the respiratory container, and the patient inhales the first gas through the patient interface.

In the embodiments of the disclosure, the patient-end respiratory system may include an inspiratory branch, and the drive gas drives the first gas in the respiratory container to be transmitted to the patient interface through the inspiratory branch connected to the respiratory container.

In the embodiments of the disclosure, the first gas is a mixed gas of air and an anesthetic gas. In the embodiments of the disclosure, the drive gas may be air, and when the drive gas is input to the intake branch of the expiratory ventilation device, it may be input according to a fixed ratio. The fixed ratio is determined according to the requirements of actual ventilation, which is not limited to the embodiments of the disclosure.

At S02, a first flow of the drive gas in the respiratory container is measured by a flow monitor.

In the embodiments of the disclosure, the flow monitor is a bidirectional flow sensor, and the flow monitor measures the first flow of the drive gas. The flow monitor may be arranged at a junction of the intake branch and the exhaust branch. The flow monitor may be a membrane flow sensor, a vane flow sensor, or a Karman vortex flow sensor, which is not limited in the embodiments of the disclosure.

At S03, at an expiratory phase, an exhaled gas is received from the patient interface and is transmitted to the respiratory container through the patient-end respiratory system, and a superfluous mixed gas in the respiratory container is exhausted through the exhaust branch.

In the embodiments of the disclosure, at the expiratory phase, the exhaled gas from the patient is transmitted from the patient-end respiratory system to the respiratory container through the patient interface. The exhaled gas compresses the mixed gas in the respiratory container out of the respiratory container, and the mixed gas is exhausted through the exhaust branch of the respiratory container.

In the embodiments of the disclosure, the mixed gas may be the drive gas, or the mixed gas may be a mixed gas of the drive gas, an anesthetic gas and the exhaled gas.

In the embodiments of the disclosure, the patient-end respiratory system may include an expiratory branch. The exhaled gas from the patient is transmitted from the expiratory branch to the respiratory container through the patient interface.

At S04, a second flow of the mixed gas is measured by the flow monitor.

In the embodiments of the disclosure, the second flow of the mixed gas exhausted by the exhaust branch is measured by the flow monitor.

In the embodiments of the disclosure, the flow monitor may include two flow sensors, which respectively measure the first flow and the second flow.

At S05, a tidal volume for one respiratory cycle is calculated on the basis of the first flow and the second flow.

In the embodiments of the disclosure, the processor uses the first flow as an inspiratory tidal volume. The processor uses the second flow as an expiratory tidal volume. The processor obtains the tidal volume for one respiratory cycle by adding the first flow to the second flow.

In the embodiments of the disclosure, the processor obtains the inspiratory tidal volume by adding the first flow to a fixed value. The processor obtains the expiratory tidal volume by subtracting the fixed value from the second flow. The processor obtains the tidal volume for one respiratory cycle by adding the first flow to the second flow after calculation. The fixed value may be a flow of a fresh gas inhaled into the lungs or into the respiratory container during respiration of a patient.

In the embodiments of the disclosure, the respiratory ventilation device may include the processor. The processor of the respiratory ventilation device adds the first flow to the second flow to obtain the tidal volume for one respiratory cycle.

In the embodiments of the disclosure, the processor may be implemented by software, hardware, firmware or a combination thereof, and may use a circuit, a single or multiple application specific integrated circuits (ASIC), a single or multiple general integrated circuits, a single or multiple microprocessors, a single or multiple programmable logic devices, or a combination of the above-mentioned circuits or devices, or other suitable circuits or devices, so that the processor can perform corresponding steps of the respiratory ventilation method.

In the embodiments of the disclosure, at the inspiratory phase, the first gas in the respiratory container is driven to the patient-end respiratory system by means of the drive gas, and is transmitted to the patient interface through the patient-end respiratory system, and the first flow of the drive gas in the respiratory container is measured by the flow monitor; at the expiratory phase, an exhaled gas is received from the patient interface and is transmitted to the respiratory container through the patient-end respiratory system, a superfluous mixed gas in the respiratory container is exhausted through the exhaust branch, and a second flow of the mixed gas is measured by the flow monitor; and the flow monitor sends the values of the first flow and the second flow to the processor, the processor may calculate the tidal volume on the basis of the first flow and the second flow. Since the flow monitor may monitor the gas flows in the machine-end respiratory system and the influence of different gas flows during inhalation and exhalation is taken into account, the tidal volume obtained by monitoring in this way has a high accuracy.

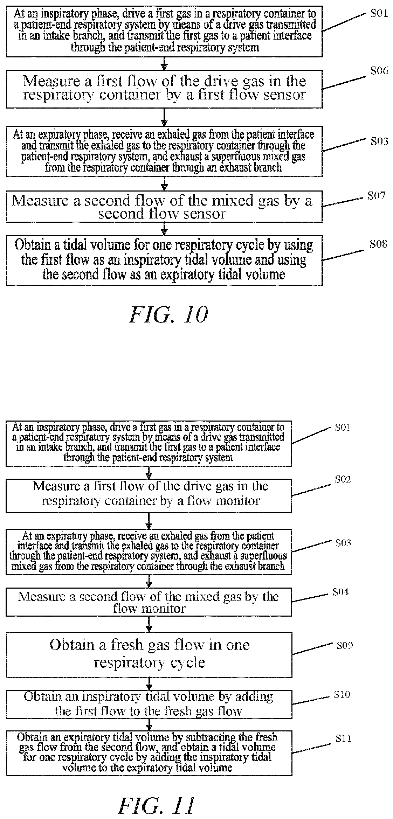

In some embodiments, referring to FIG. 10, FIG. 10 is a schematic flow chart of an optional respiratory ventilation method according to the embodiments of the disclosure. S02 shown in FIG. 9 may be implemented by S06 and will be described in conjunction with various steps.

At S06, a first flow of the drive gas in the respiratory container is measured by a first flow sensor.

In the embodiments of the disclosure, the flow monitor includes a first flow sensor and a second flow sensor. The first flow sensor and the second flow sensor are one-way flow sensors. The first flow sensor is connected to the intake branch. At the inspiratory phase, the first flow of the drive gas in the respiratory container is measured by the first flow sensor.

In the embodiments of the disclosure, the intake branch and the exhaust branch of the machine-end respiratory system are respectively connected to the respiratory container via the first flow sensor and the second flow sensor. At the inspiratory phase, the first flow of the drive gas in the respiratory container is measured by the first flow sensor.

The first flow sensor may be connected to the first one-way valve, and the second flow sensor may be connected to the second one-way valve. The first one-way valve is configured to transmit the drive gas to the respiratory container; and the second one-way valve is configured to transmit the mixed gas to the exhaust branch.

In some embodiments, referring to FIG. 10, FIG. 10 is a schematic flow chart of an optional respiratory ventilation method according to the embodiments of the disclosure. S04 and S05 shown in FIG. 9 may be implemented by S07 and S08 and will be described in conjunction with various steps.

At S07, a second flow of the mixed gas is measured by the second flow sensor.

In the embodiments of the disclosure, the flow monitor includes a first flow sensor and a second flow sensor. The first flow sensor and the second flow sensor are one-way flow sensors. The second flow sensor is connected to the exhaust branch. At the expiratory phase, the second flow of the mixed gas is measured by the second flow sensor.

At S08, the tidal volume for one respiratory cycle is obtained by using the first flow as an inspiratory tidal volume and using the second flow as an expiratory tidal volume.

In the embodiments of the disclosure, the processor uses the first flow as the inspiratory tidal volume. The processor uses the second flow as the expiratory tidal volume. The processor obtains the tidal volume for one respiratory cycle by adding the first flow to the second flow.

In the embodiments of the disclosure, at the inspiratory phase, the first gas in the respiratory container is driven to the patient-end respiratory system by means of the drive gas, and is transmitted to the patient interface through the patient-end respiratory system, and the first flow of the drive gas in the respiratory container is measured by the first flow sensor; at the expiratory phase, an exhaled gas is received from the patient interface and is transmitted to the respiratory container through the patient-end respiratory system, a superfluous mixed gas in the respiratory container is exhausted through the exhaust branch, and a second flow of the mixed gas is measured by the second flow sensor; and the first flow sensor sends the value of the first flow to the processor, the second flow sensor sends the value of the second flow to the processor, and the processor may calculate the tidal volume on the basis of the first flow and the second flow. Since the flow monitor may monitor the gas flows in the machine-end respiratory system and the influence of different gas flows during inhalation and exhalation is taken into account, the tidal volume obtained by monitoring in this way has a high accuracy.

In some embodiments, referring to FIG. 11, FIG. 11 is a schematic flow chart of an optional respiratory ventilation method according to the embodiments of the disclosure. S05 shown in FIG. 9 may be implemented by S09-S 11 and will be described in conjunction with various steps.

At S09, a fresh gas flow in one respiratory cycle is obtained.

In the embodiments of the disclosure, the fresh gas flow in one respiratory cycle may be measured by the flow sensor.

In the embodiments of the disclosure, the fresh gas flow in one respiratory cycle may be a difference between the fresh gas flow inhaled into the lungs of a patient during inhalation of the patient and the fresh gas flow inhaled into the respiratory container during exhalation of the patient.

In the embodiments of the disclosure, the fresh gas flow in one respiratory cycle may be manually input by means of a keyboard or an input device provided on the respiratory ventilation device.

At S 10, an inspiratory tidal volume is obtained by adding the first flow to the fresh gas flow.

In the embodiments of the disclosure, the processor obtains the inspiratory tidal volume by adding the first flow to the fresh gas flow.

In the embodiments of the disclosure, the first flow is the flow of the first gas input into the patient interface from the respiratory container, and the fresh gas flow is the flow of the third gas inhaled by the patient during inhalation.

At S 11, an expiratory tidal volume is obtained by subtracting the fresh gas flow from the second flow, and the tidal volume for one respiratory cycle is obtained by adding the inspiratory tidal volume to the expiratory tidal volume.

In the embodiments of the disclosure, the processor obtains the expiratory tidal volume by subtracting the fresh gas flow from the second flow. The processor obtains the tidal volume for one respiratory cycle by adding the inspiratory tidal volume to the expiratory tidal volume.

In the embodiments of the disclosure, the second flow is the flow of the mixed gas exhausted from the respiratory container during exhalation of the patient. The fresh gas flow is the flow of the fifth gas entering the respiratory container during the exhalation of the patient.

In the embodiments of the disclosure, the processor is provided with a flow monitor on one side of the machine-end respiratory system, and the flow monitor is away from the patient. At the inspiratory phase, the first gas in the respiratory container is driven to the patient-end respiratory system by means of the drive gas, and is transmitted to the patient interface through the patient-end respiratory system, and the first flow of the drive gas in the respiratory container is measured by the flow monitor; and the fresh gas flow is measured by the flow sensor at the same time. At the expiratory phase, the exhaled gas is received from the patient interface and is transmitted to the respiratory container through the patient-end respiratory system, and a fresh gas is received at the same time. With regard to the mixed gas in the respiratory container, the superfluous mixed gas is exhausted under the action of the fresh gas and the exhaled gas, the second flow of the exhausted mixed gas is measured by the flow monitor, and the fresh gas flow is measured by the flow sensor. The processor may calculate the tidal volume on the basis of the first flow, the second flow and the fresh gas flow at the respiratory phase. Since the flow monitor may monitor the gas flows in the machine-end respiratory system and the influence of different gas flows during inhalation and exhalation is taken into account, the tidal volume obtained by monitoring in this way has a high accuracy.

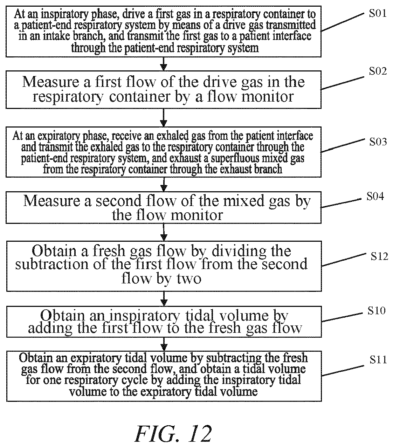

In some embodiments, referring to FIG. 12, FIG. 12 is a schematic flow chart of an optional respiratory ventilation method according to the embodiments of the disclosure. S09 shown in FIG. 11 may be implemented by S 12 and will be described in conjunction with various steps.

At S12, the fresh gas flow is obtained by dividing the subtraction of the first flow from the second flow by two.

In the embodiments of the disclosure, at the inspiratory phase, the first flow is only the first gas flow during inhalation of the patient, and the second gas flow further includes the exhaled gas flow and the fresh gas flow during the exhalation of the patient. The processor obtains the sum of the fresh gas flows of the patient during the inhalation and the exhalation by subtracting the first flow from the second flow. Moreover, since the fresh gas flows of the patient during the inhalation and the exhalation are the same, the processor may obtain the fresh gas flow of the patient during the inhalation or the exhalation by dividing the sum of the fresh gas flows by 2.

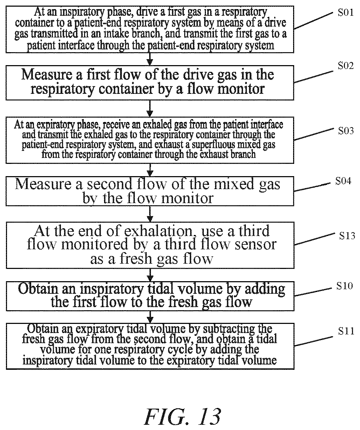

In some embodiments, referring to FIG. 13, FIG. 13 is a schematic flow chart of an optional respiratory ventilation method according to the embodiments of the disclosure. S09 shown in FIG. 11 may be implemented by S 13 and will be described in conjunction with various steps.

At S13, at the end of exhalation, a third flow monitored by the third flow sensor is used as the fresh gas flow.

In the embodiments of the disclosure, at the respiratory phase, since the flow of the fresh gas actually entering the respiratory ventilation device is very small, the patient's exhalation has completely ended at the end of the expiratory phase. In this case, the gas bag has risen to the top, the fresh gas continuing to enter the gas bag is mixed with the gas in the gas bag, and when the pressure in the gas bag is greater than the pressure threshold of the gas valve connected to the gas bag, the mixed gas in the gas bag completely overflows from the gas valve. The flow of the gas overflowing from the gas valve is the same as the flow of the fresh gas. Therefore, at the end of exhalation, the fresh gas flow may be obtained on the basis of the data monitored by the third flow sensor in a point measurement manner.

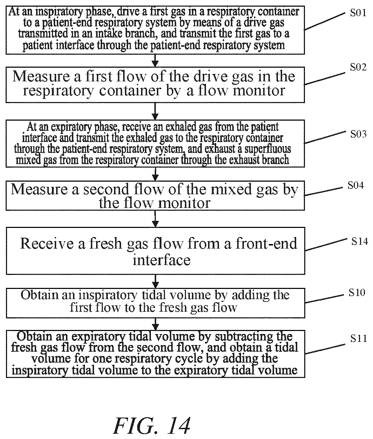

In some embodiments, referring to FIG. 14, FIG. 14 is a schematic flow chart of an optional respiratory ventilation method according to the embodiments of the disclosure. S09 shown in FIG. 11 may be implemented by S 14 and will be described in conjunction with various steps.

At S14, a fresh gas flow is received from a front-end interface.

In the embodiments of the disclosure, the front-end interface of the respiratory ventilation device may be displayed on a touch screen. Moreover, the respiratory ventilation device may further include an input keyboard. An operator may input a flow value of the fresh gas by means of the input keyboard or the touch screen. The processor obtains the fresh gas flow according to the input value. The input value is then the fresh gas flow.

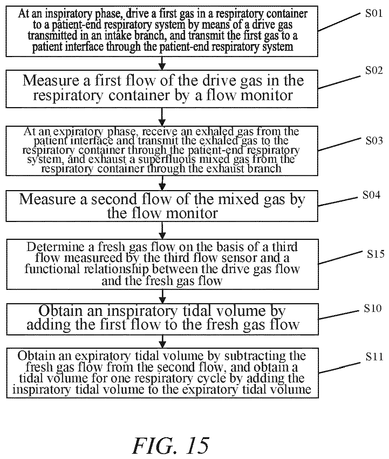

In some embodiments, referring to FIG. 15, FIG. 15 is a schematic flow chart of an optional respiratory ventilation method according to the embodiments of the disclosure. S09 shown in FIG. 11 may be implemented by S 15 and will be described in conjunction with various steps.

At S15, a fresh gas flow is determined on the basis of the third flow measured by the third flow sensor and a functional relationship between the drive gas flow and the fresh gas flow.

In the embodiments of the disclosure, operating times at the inspiratory phase and at the expiratory phase are the same, and there is a corresponding functional relationship between intake volumes of a drive gas inlet and a fresh gas inlet. Therefore, the processor may determine the fresh gas flow on the basis of the functional relationship between the drive gas flow and the fresh gas flow.

In the embodiments of the disclosure, the drive gas flow may be the fresh gas flow multiplied by N. N may be 1, 2, 10 or 0.2, and the specific value thereof is not limited.

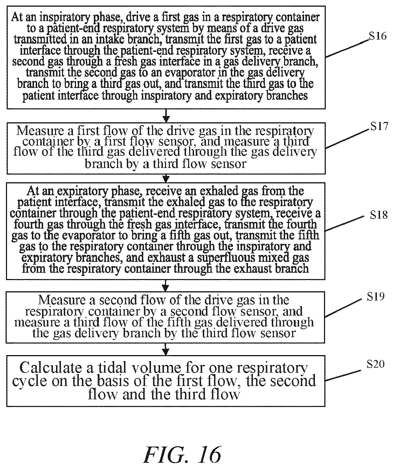

On the basis of the structure of the respiratory ventilation device described above, FIG. 16 is a schematic flow chart of a respiratory ventilation method applied to a respiratory ventilation device according to the embodiments of the disclosure. As shown in FIG. 16, the respiratory ventilation method mainly includes the following steps.

At S 16, at an inspiratory phase, a first gas in a respiratory container is driven to a patient-end respiratory system by means of a drive gas transmitted in an intake branch, and is transmitted to a patient interface through the patient-end respiratory system, a second gas is received through a fresh gas interface in a gas delivery branch and is transmitted to an evaporator in the gas delivery branch to bring a third gas out, and the third gas is transmitted to the patient interface through inspiratory and expiratory branches.

In the embodiments of the disclosure, at the inspiratory phase, the intake branch of the machine-end respiratory system transmits the drive gas to the respiratory container. The drive gas drives the first gas in the respiratory container to be transmitted to the patient interface through the inspiratory branch connected to the respiratory container, and the patient inhales the first gas through the patient interface. At the same time, the second gas is received through the fresh gas interface and is transmitted to the evaporator, the second gas brings the third gas out of the evaporator, and the third gas is transmitted to the patient interface through the inspiratory branch.

In the embodiments of the disclosure, the second gas may be air. The third gas may be a mixed gas of an anesthetic gas and air.

At S17, a first flow of the drive gas in the respiratory container is measured by the first flow sensor, and a third flow of the third gas delivered through the gas delivery branch is measured by the third flow sensor.

In the embodiments of the disclosure, the first flow sensor measures the first flow of the drive gas. The third flow sensor measures the third flow of the third gas delivered through the gas delivery branch.

In the embodiments of the disclosure, the first flow sensor may be arranged at the intake branch. The first flow sensor may also be arranged at a junction of the intake branch and the exhaust branch. The third flow sensor is arranged at the gas delivery branch.

At S 18, at an expiratory phase, an exhaled gas is received from the patient interface and is transmitted to the respiratory container through the patient-end respiratory system, a fourth gas is received through the fresh gas interface and is transmitted to the evaporator to bring a fifth gas out, and the fifth gas is transmitted to the respiratory container through the inspiratory and expiratory branches, and a superfluous mixed gas in the respiratory container is exhausted through the exhaust branch.

In the embodiments of the disclosure, at the expiratory phase, the exhaled gas from the patient is transmitted from the patient-end respiratory system to the respiratory container through the patient interface. Furthermore, the fourth gas is received through the fresh gas interface and is transmitted to the evaporator, the fourth gas brings the fifth gas out of the evaporator, and the fifth gas is transmitted to the respiratory container through the patient-end respiratory system. The exhaled gas and the fifth gas compress the superfluous mixed gas in the respiratory container out of the respiratory container, and the superfluous mixed gas is exhausted through the exhaust branch.

At S19, the second flow of the mixed gas is measured by the second flow sensor, and the third flow of the fifth gas delivered through the gas delivery branch is measured by the third flow sensor.

In the embodiments of the disclosure, the second flow of the mixed gas exhausted by the exhaust branch is measured by the second flow sensor. The third flow of the fifth gas delivered through the gas delivery branch is measured by the third flow sensor.

At S20, a tidal volume for one respiratory cycle is calculated on the basis of the first flow, the second flow and the third flow.

In the embodiments of the disclosure, at the inspiratory phase, the processor obtains the inspiratory tidal volume at the inspiratory phase by adding the first flow to the third flow of the third gas. At the expiratory phase, the processor obtains the expiratory tidal volume at the expiratory phase by subtracting the third flow of the fifth gas from the second flow. In the embodiments of the disclosure, the inspiratory tidal volume plus the expiratory tidal volume equals the tidal volume for one respiratory cycle.

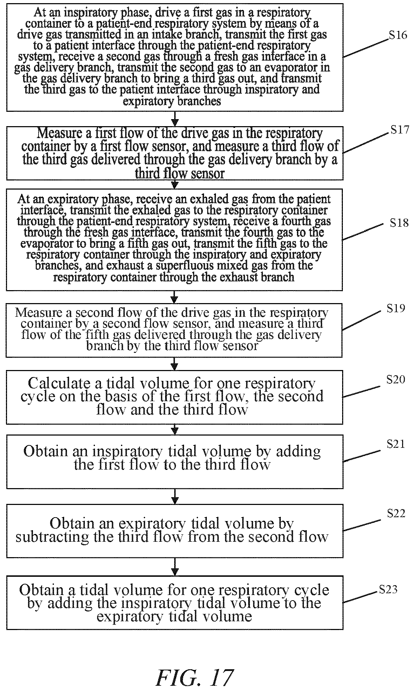

In some embodiments, referring to FIG. 17, FIG. 17 is a schematic flow chart of an optional respiratory ventilation method according to the embodiments of the disclosure. S20 shown in FIG. 16 may be implemented by S21-S23 and will be described in conjunction with various steps.

At S21, an inspiratory tidal volume is obtained by adding the first flow to the third flow.

In the embodiments of the disclosure, the processor obtains the inspiratory tidal volume by adding the first flow to the third flow.

The first flow is the drive gas flow. The third flow is the fresh gas flow during the inhalation.

At S22, an expiratory tidal volume is obtained by subtracting the third flow from the second flow.

In the embodiments of the disclosure, the processor obtains the expiratory tidal volume by subtracting the third flow of the fifth gas from the second flow.

At S23, a tidal volume for one respiratory cycle is obtained by adding the inspiratory tidal volume to the respiratory tidal volume.

In the embodiments of the disclosure, the processor obtains the tidal volume for one respiratory cycle by adding the inspiratory tidal volume to the expiratory tidal volume.

In the embodiments of the disclosure, the flow monitor is arranged on one side of the machine-end respiratory system, and the flow monitor is away from the patient. At the inspiratory phase, the first gas in the respiratory container is driven to the patient-end respiratory system by means of the drive gas, and is transmitted to the patient interface through the patient-end respiratory system, and the first flow of the drive gas in the respiratory container is measured by the flow monitor. At the same time, the third gas is transmitted through the gas transmission branch and is transmitted to the patient interface through the patient-end respiratory system, and the third flow of the third gas is measured by the third flow sensor. At the expiratory phase, the exhaled gas is received from the patient interface and is transmitted to the respiratory container through the patient-end respiratory system, and the fifth gas is received from the gas delivery branch at the same time and is transmitted to the respiratory container through the patient-end respiratory system. Under the action of the fifth gas and the exhaled gas for the respiratory container, the superfluous mixed gas in the respiratory container is exhausted through the exhaust branch, the second flow of the exhausted mixed gas is measured by the flow monitor, and a fourth flow of the fifth gas delivered through the gas delivery branch is measured by the third flow sensor. The processor may calculate the tidal volume on the basis of the first flow, the second flow and the third flow. Since the flow monitor may monitor the gas flows in the machine-end respiratory system and the influence of different gas flows during inhalation and exhalation is taken into account, the tidal volume obtained by monitoring in this way has a high accuracy.

The embodiments of the disclosure provide an anesthesia machine, including a respiratory ventilation device of the structure described above.

The embodiments of the disclosure further provide a computer-readable storage medium storing executable ventilation instructions configured to implement, when executed by a processor of a respiratory ventilation device, a respiratory ventilation method according to the embodiments of the disclosure.

Various components in the embodiments of the disclosure may be integrated into one processing unit, or various units may be physically present separately, or two or more units may be integrated into one unit. The integrated units described above may be implemented in the form of hardware or software functional modules.

If the integrated units are implemented in the form of the software functional modules but not sold or used as independent products, they may be stored in a computer-readable storage medium. Based on such an understanding, the technical solution of this embodiment, in essence, or its part contributing to the prior art, or all or part of the technical solution may be embodied in the form of a software product. The computer software product is stored in a storage medium and includes a number of instructions for causing a computer device (which may be a personal computer, a server, or a network device) or a processor to execute all or some of the steps of the method according to this embodiment. The foregoing storage medium includes various media that can store program codes, such as a ferromagnetic random access memory (FRAM), a Read Only Memory (ROM), a Programmable Read-Only Memory (PROM), an EPROM (Erasable Programmable Read-Only Memory), an EEPROM (Electrically Erasable Programmable Read-Only Memory), a Flash Memory, a magnetic surface memory, a compact disc, or a Compact Disc Read-Only Memory (CD-ROM), which is not limited in the embodiments of the disclosure.

### INDUSTRIAL APPLICABILITY

Embodiments of the disclosure provide a respiratory ventilation method and device, an anesthesia machine, and a computer-readable storage medium. A flow monitor is provided on one side of a machine-end respiratory system in the respiratory ventilation device and is away from a patient end. At an inspiratory phase, a first gas in a respiratory container is driven to a patient-end respiratory system by means of a drive gas, and is transmitted to a patient interface through the patient-end respiratory system, and a first flow of the drive gas in the respiratory container is measured by the flow monitor; at an expiratory phase, an exhaled gas is received from the patient interface and is transmitted to the respiratory container through the patient-end respiratory system, a superfluous mixed gas in the respiratory container is exhausted through an exhaust branch, and a second flow of the mixed gas is measured by the flow monitor; and the processor calculates a tidal volume for one respiratory cycle on the basis of the first flow and the second flow. Since the flow monitor may monitor the gas flows in the machine-end respiratory system and the influence of different gas flows during inhalation and exhalation is taken into account, the tidal volume obtained by monitoring in this way has a high accuracy.

## Claims

1. A respiratory ventilation device, **characterized in that** the respiratory ventilation device comprises: a patient-end respiratory system, a machine-end respiratory system, a flow monitor and a processor, wherein
the machine-end respiratory system comprises: an intake branch at an inspiratory phase, a respiratory container, and an exhaust branch at an expiratory phase;
the intake branch and the exhaust branch are both connected to the respiratory container via the flow monitor, and the respiratory container is connected to the patient-end respiratory system;
the processor is connected to the flow monitor; wherein
at the inspiratory phase, a first gas in the respiratory container is driven to the patient-end respiratory system by means of a drive gas transmitted in the intake branch, and is transmitted to a patient interface through the patient-end respiratory system, and a first flow of the drive gas in the respiratory container is measured by the flow monitor;
at the expiratory phase, an exhaled gas is received from the patient interface and is transmitted to the respiratory container through the patient-end respiratory system, a superfluous mixed gas in the respiratory container is exhausted through the exhaust branch, and a second flow of the mixed gas is measured by the flow monitor; and
the processor is configured to calculate a tidal volume for one respiratory cycle on the basis of the first flow and the second flow.

2. The respiratory ventilation device of claim 1, **characterized in that** the flow monitor is a bidirectional flow sensor.

3. The respiratory ventilation device of claim 1, **characterized in that** the flow monitor is a one-way flow sensor; the flow monitor comprises: a first flow sensor and a second flow sensor;
the first flow sensor is connected to the intake branch, and the second flow sensor is connected to the exhaust branch;
at the inspiratory phase, the first flow of the drive gas in the respiratory container is measured by the first flow sensor; and
at the expiratory phase, the second flow of the mixed gas is measured by the second flow sensor.

4. The respiratory ventilation device of claim 1, **characterized in that** the respiratory ventilation device further comprises: a first one-way valve and a second one-way valve; the flow monitor is a one-way flow sensor; the flow monitor comprises: a first flow sensor and a second flow sensor;
the first one-way valve is configured to transmit the drive gas to the respiratory container;
the second one-way valve is configured to transmit the mixed gas to the exhaust branch;
the intake branch and the exhaust branch are both connected to the respiratory container via the first flow sensor, the first one-way valve, the second flow sensor and the second one-way valve; the first one-way valve is connected to the first flow sensor, and the second one-way valve is connected to the second flow sensor;
at the inspiratory phase, the first flow of the drive gas in the respiratory container is measured by means of the first flow sensor and the first one-way valve; and
at the expiratory phase, the second flow of the mixed gas is measured by means of the second flow sensor and the second one-way valve.

5. The respiratory ventilation device of any one of claims 1-4, **characterized in that** the respiratory ventilation device further comprises: a gas delivery branch; the gas delivery branch is provided with a third flow sensor, a fresh gas interface and an evaporator;
at the inspiratory phase, a second gas is received through the fresh gas interface and is transmitted to the evaporator to bring a third gas out, and the third gas is transmitted to the patient interface through the patient-end respiratory system; a third flow of the third gas delivered through the gas delivery branch is measured by the third flow sensor;
at the expiratory phase, a fourth gas is received through the fresh gas interface and is transmitted to the evaporator to bring a fifth gas out, and the fifth gas is transmitted to the respiratory container through the patient-end respiratory system; a third flow of the fifth gas delivered through the gas delivery branch is measured by the third flow sensor; and
the processor is configured to calculate a tidal volume for one respiratory cycle on the basis of the first flow, the second flow and the third flow.

6. The respiratory ventilation device of claim 5, **characterized in that** the patient-end respiratory system further comprises: an inspiratory branch and an expiratory branch;
the inspiratory branch and the expiratory branch are both connected to the respiratory container at one common connection end of the inspiratory branch and the expiratory branch, the gas delivery branch is connected to the inspiratory branch, and the patient interface is provided at the other common connection end of the inspiratory branch and the expiratory branch;
at the inspiratory phase, the drive gas is transmitted to the inspiratory branch through a drive gas interface, and the first gas in the respiratory container is driven to the inspiratory branch and is transmitted from the inspiratory branch to the patient interface; and
at the expiratory phase, the exhaled gas is received from the patient interface and is transmitted to the respiratory container through the expiratory branch, and the superfluous mixed gas in the respiratory container is exhausted through the exhaust branch.

7. The respiratory ventilation device of claim 5, **characterized in that** the respiratory container comprises: a gas mask, a gas bag and a gas valve, wherein
the gas bag is arranged in an inner cavity of the gas mask and is connected to the patient-end respiratory system, the gas bag is of a deformable structure, and the gas bag isolates the drive gas in the gas mask from the first gas in the gas bag;
the intake branch is connected to the gas mask, and the exhaust branch is connected to the gas bag via the gas valve;
at the inspiratory phase, the drive gas is transmitted to the gas mask through the intake branch, the gas bag shrinks and deforms under the action of a drive gas pressure in the gas mask, the first gas is compressed to be transmitted to the patient interface through the patient-end respiratory system, and the third gas is transmitted through the gas transmission branch and is transmitted to the patient interface through the patient-end respiratory system;
at the expiratory phase, the exhaled gas is received from the patient interface and is transmitted to the gas bag through the patient-end respiratory system, and the fifth gas is received from the gas delivery branch and is transmitted to the gas bag through the patient-end respiratory system; and
the gas bag expands and deforms under the action of the fifth gas and the exhaled gas, the drive gas in the gas mask is compressed to be exhausted through the exhaust branch, and when a pressure in the gas bag is higher than a pressure threshold of the gas valve, the fifth gas and the exhaled gas in the gas bag are compressed to be exhausted through the exhaust branch.

8. The respiratory ventilation device of claim 5, **characterized in that** the respiratory container comprises: an exchange cavity;
the exchange cavity is of a hollow tubular structure, one end of the exchange cavity is connected to the intake branch and the exhaust branch, and the other end of the exchange cavity is connected to the patient-end respiratory system;
at the inspiratory phase, the drive gas is transmitted to the exchange cavity through the intake branch, the first gas in the exchange cavity is transmitted to the patient interface through the patient-end respiratory system under a pushing action of the drive gas pressure, and the third gas is transmitted through the gas transmission branch and is transmitted to the patient interface through the patient-end respiratory system;
at the expiratory phase, the exhaled gas is received from the patient interface and is transmitted to the exchange cavity through the patient-end respiratory system, and the fifth gas is received from the gas delivery branch and is transmitted to the exchange cavity through the patient-end respiratory system; and
the fifth gas and the exhaled gas in the exchange cavity push the drive gas for mixing, to form the mixed gas, and the mixed gas is exhausted through the exhaust branch.

9. A respiratory ventilation method, **characterized in that** the method is applied to a respiratory ventilation device of any one of claims 1-9, and comprises:
at an inspiratory phase, driving a first gas in a respiratory container to a patient-end respiratory system by means of a drive gas transmitted in an intake branch, and transmitting the first gas to a patient interface through the patient-end respiratory system;
measuring a first flow of the drive gas in the respiratory container by a flow monitor;
at an expiratory phase, receiving an exhaled gas from a patient interface and transmitting the exhaled gas to the respiratory container through the patient-end respiratory system, and exhausting a superfluous mixed gas from the respiratory container through an exhaust branch;
measuring a second flow of the mixed gas by the flow monitor; and
calculating a tidal volume for one respiratory cycle on the basis of the first flow and the second flow.

10. The respiratory ventilation method of claim 9, **characterized in that** the flow monitor comprises: a first flow sensor and a second flow sensor;
measuring a first flow of the drive gas in the respiratory container by a flow monitor comprises:
measuring the first flow of the drive gas in the respiratory container by the first flow sensor; and
measuring a second flow of the mixed gas by the flow monitor comprises:
measuring the second flow of the mixed gas by the second flow sensor.

11. The respiratory ventilation method of claim 9 or 10, **characterized in that** calculating a tidal volume for one respiratory cycle on the basis of the first flow and the second flow comprises:
obtaining the tidal volume for one respiratory cycle by using the first flow as an inspiratory tidal volume and using the second flow as an expiratory tidal volume.

12. The respiratory ventilation method of claim 9 or 10, **characterized in that** calculating a tidal volume for one respiratory cycle on the basis of the first flow and the second flow comprises:
obtaining a fresh gas flow in one respiratory cycle;
obtaining an inspiratory tidal volume by adding the first flow to the fresh gas flow; and
obtaining an expiratory tidal volume by subtracting the fresh gas flow from the second flow, and obtaining the tidal volume for one respiratory cycle by adding the inspiratory tidal volume to the expiratory tidal volume.

13. The respiratory ventilation method of claim 12, **characterized in that** the respiratory ventilation device comprises: a third flow sensor; obtaining a fresh gas flow in one respiratory cycle comprises:
obtaining the fresh gas flow by dividing the subtraction of the first flow from the second flow by two; or
at the end of exhalation, using a third flow monitored by the third flow sensor as the fresh gas flow; or
receiving the fresh gas flow from a front-end interface; or
determining the fresh gas flow on the basis of a third flow measured by the third flow sensor and a functional relationship between a drive gas flow and the fresh gas flow.

14. The respiratory ventilation method of claim 9 or 10, **characterized in that** the patient-end respiratory system comprises a gas delivery branch; the method further comprises:
at the inspiratory phase, receiving a second gas through a fresh gas interface in the gas delivery branch and transmitting the second gas to a evaporator in the gas delivery branch to bring a third gas out, and transmitting the third gas to the patient interface through the patient-end respiratory system;
measuring, by the third flow sensor, a third flow of the third gas delivered through the gas delivery branch;
at the expiratory phase, receiving a fourth gas through the fresh gas interface and transmitting the fourth gas to the evaporator to bring a fifth gas out, and transmitting the fifth gas to the respiratory container through the patient-end respiratory system; and
measuring, by the third flow sensor, a third flow of the fifth gas delivered through the gas delivery branch.

15. The respiratory ventilation method of claim 14, **characterized in that** calculating a tidal volume for one respiratory cycle on the basis of the first flow and the second flow comprises:
calculating a tidal volume for one respiratory cycle on the basis of the first flow, the second flow and the third flow.

16. The respiratory ventilation method of claim 15, **characterized in that** calculating a tidal volume for one respiratory cycle on the basis of the first flow, the second flow and the third flow comprises:
obtaining an inspiratory tidal volume by adding the first flow to the third flow;
obtaining an expiratory tidal volume by subtracting the third flow from the second flow; and
obtaining the tidal volume for one respiratory cycle by adding the inspiratory tidal volume to the respiratory tidal volume.

17. An anesthesia machine, **characterized in that** the anesthesia machine comprises:
a respiratory ventilation device of any one of claims 1-8.

18. A computer-readable storage medium, **characterized in that** the computer-readable storage medium stores executable instructions configured to implement, when executed by a processor of an expiratory ventilation device, a method of any one of claims 9-16.
